# FASCICULE DE BREVET EUROPEEN

(11) **EP 1 000 063 B1**
(45) Date de publication et mention de la délivrance du brevet: **23.10.2002**
(21) Numéro de dépôt: 98941468.5
(22) Date de dépôt: 28.07.1998
(51) Int. Cl.: C07D 487/04, A61K 31/50

(54) **DERIVES DE 4-OXO-3,5-DIHYDRO-4H-PYRIDAZINO[4,5-b]INDOLE-1-ACETAMIDE, LEUR PREPARATION ET LEUR APPLICATION EN THERAPEUTIQUE**
4-OXO-3,5-DIHYDRO-4H-PYRIDAZINO[4,5-b]INDOL-1-ACETAMID-DERIVATE, IHRE HERSTELLUNG UND THERAPEUTISCHE ANWENDUNG
4-OXO-3,5-DIHYDRO-4H-PYRIDAZINO[4,5-b]INDOLE-1-ACETAMIDE DERIVATIVES, THEIR PREPARATION AND THEIR APPLICATION IN THERAPY

(30) Priorité: 30.07.1997 FR 9709692
(43) Date de publication de la demande: 17.05.2000
(73) Titulaire: SANOFI-SYNTHELABO, 75013 Paris (FR)
(72) Inventeur: EVANNO, Yannick, F-78830 Bullion (FR); DUBOIS, Laurent, F-91190 Gif sur Yvette (FR); SEVRIN, Mireille, F-75014 Paris (FR); MARGUET, Frank, F-91370 Verrières le Buisson (FR); FROISSANT, Jacques, F-4116 0 Morée (FR); BARTSCH, Régine, F-92260 Fontenay aux Roses (FR); GILLE, Catherine, F-91160 Longjumeau (FR)
(74) Mandataire: Ludwig, Jacques
(86) Numéro de dépôt international: FR9801667
(87) Numéro de publication internationale: WO99006406

(56) Documents cités:
- WO-A-98/15552
- GB-A- 2 290 292

## Description

La présente invention a pour objet des composés de formule générale (I) 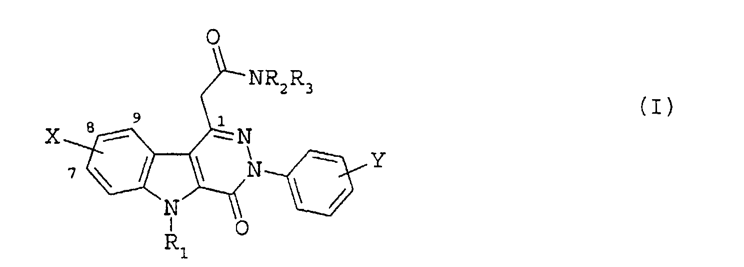 dans laquelle
X représente un atome d'hydrogène ou d'halogène ou un groupe méthyle, méthoxy ou phénylméthoxy,
Y représente un atome d'hydrogène, un ou deux atomes d'halogène ou un groupe méthyle, hydroxy, méthoxy ou nitro,
R₁ représente un atome d'hydrogène ou un groupe (C₁-C₄)alkyle,
R₂ et R₃ représentent chacun, indépendamment l'un de l'autre, un atome d'hydrogène, un groupe (C₁-C₄)alkyle ou un groupe phénylméthyle,
ou bien R₂ et R₃ forment, avec l'atome d'azote qui les porte, un groupe azétidinyle, pyrrolidinyle, 3-éthoxypyrrolidinyle, pipéridinyle, morpholinyle, 4-méthylpipérazinyle ou 1,3-thiazolidinyle, dont les formules respectives sont les suivantes :

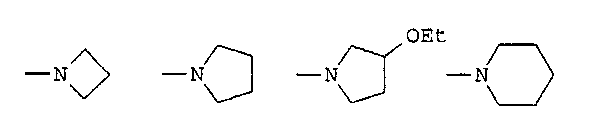

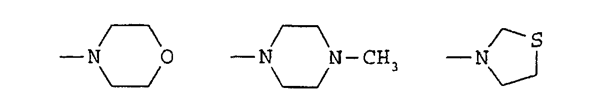

Les composés préférés sont ceux dans la formule générale desquels X est en position 8 ou 9 et représente un atome d'hydrogène ou d'halogène, Y représente un atome d'hydrogène, R₁ représente un groupe méthyle ou éthyle, R₂ représente un atome d'hydrogène ou un groupe méthyle, R₃ représente un groupe méthyle, ou bien R₂ et R₃ forment, avec l'atome d'azote qui les porte, un cycle azétidinyle ou pyrrolidinyle.

Les composés de formule générale (I) peuvent être préparés par des procédés illustrés par les schémas qui suivent.

Selon le schéma 1 on fait réagir un composé de formule générale (II), dans laquelle X et R₁ sont tels que définis ci-dessus et R'représente un groupe (C₁-C₄)alkyle, avec le chlorure d'oxalyle, dans un solvant aprotique tel que le toluène, entre 50°C et la température de reflux, puis on traite l'intermédiaire réactionnel à température ambiante par un alcool de formule générale R"OH dans laquelle R" représente un groupe (C₁-C₄)alkyle pour obtenir le diester de formule générale (III), ou bien on fait réagir le composé de formule générale (II) avec un chloroglyoxylate d'alkyle dans un solvant aprotique polaire tel que le dichlorométhane, à température ambiante, en présence d'un acide de Lewis, par exemple le tétrachlorure de titane, pour obtenir le diester de formule générale (III).
On traite ensuite ce dernier dans l'acide acétique, d'abord à température ambiante puis à la température de reflux, avec une phénylhydrazine éventuellement substituée par un groupe Y tel que défini ci-dessus pour obtenir un ester de formule générale (IV). Lorsque R₁ représente un groupe alkyle on transforme cet ester en alcool correspondant, de formule générale (V), par réduction au moyen d'un agent réducteur tel que le borohydrure de sodium, dans un solvant tel que le tétrahydrofurane, en présence d'un alcool, par exemple le méthanol.

On transforme ensuite cet alcool (V) en composé halogéné de formule générale (VIII) par toute réaction connue de l'homme du métier, soit, par exemple, le traitement par le tétrabromure de carbone, en présence de triphénylphosphine, dans un solvant tel que le dichlorométhane, ou bien par action d'un agent chlorurant tel que le chlorure de méthanesulfonyle, dans un mélange de solvants tels que le tétrahydrofurane et la pyridine.

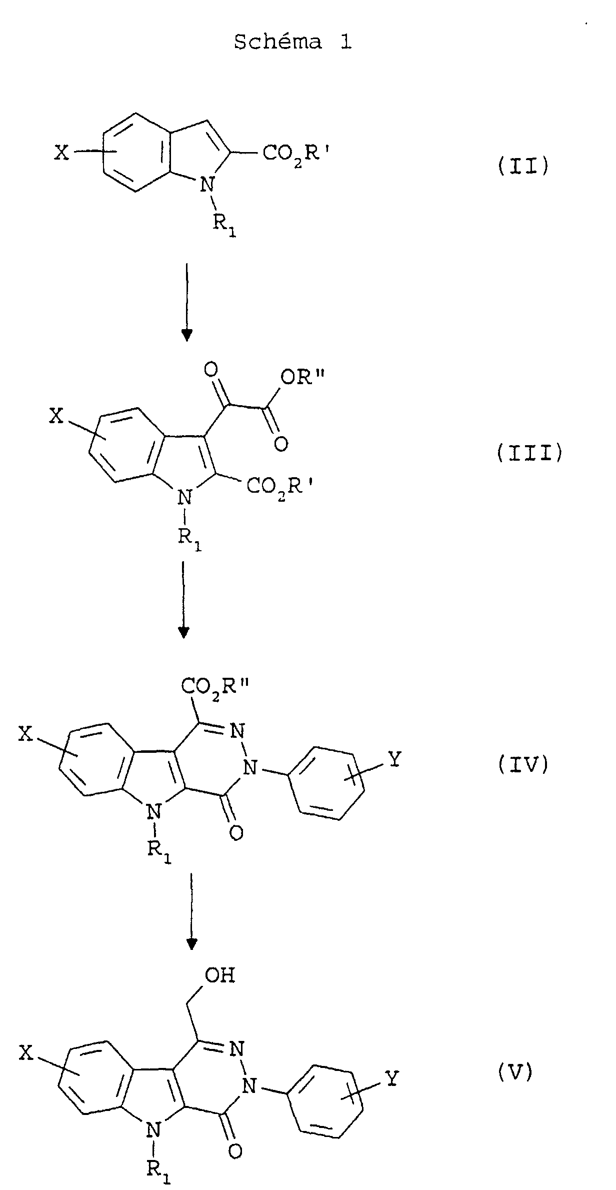

On effectue ensuite une réaction de substitution nucléophile par l'ion cyanure, soit dans un mélange de solvants polaires tels que le diméthylformamide et l'eau, à une température de 20 à 80°C, soit dans un mélange biphasique, tel que l'eau et le dichlorométhane, entre la température ambiante et la température de reflux, en présence d'un agent de transfert de phase, pour obtenir le composé de formule générale (IX).

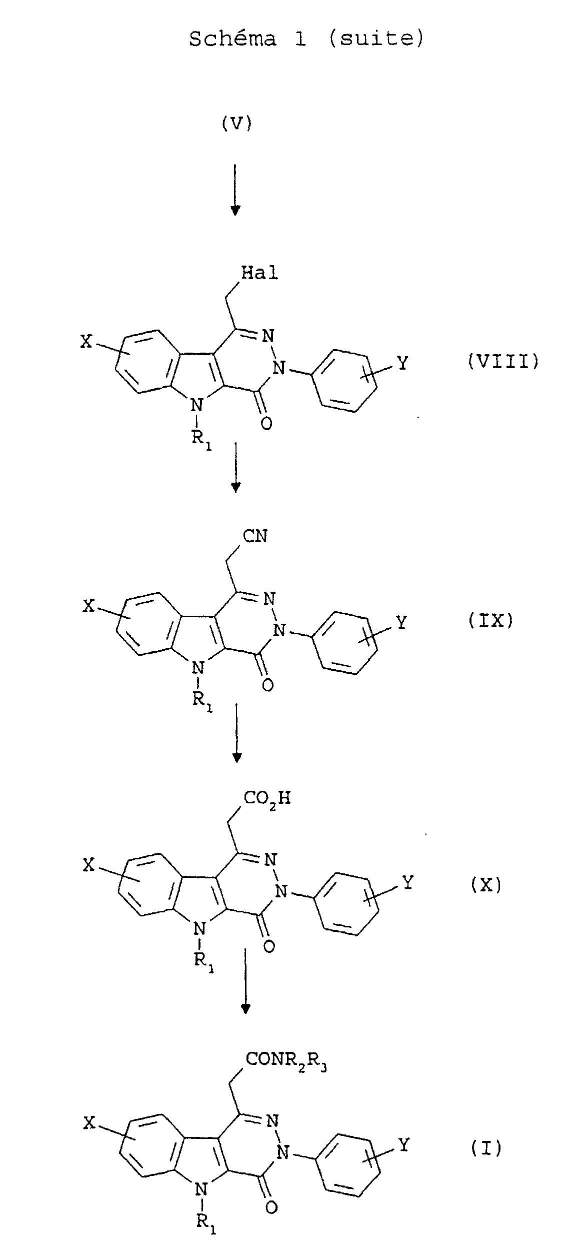

On effectue ensuite une hydrolyse acide, par exemple en utilisant un mélange d'acide acétique et d'acide chlorhydrique, à la température de reflux, ou bien une hydrolyse basique, par exemple en utilisant la potasse dans un mélange de solvants tels que l'eau et le 2-méthoxyéthanol, à la température de reflux, pour obtenir le composé de formule générale (X).
On transforme ensuite cet acide en amide secondaire ou tertiaire de formule générale (I) par action d'une amine de formule générale HNR₂R₃, dans laquelle R₂ et R₃ sont tels que définis ci-dessus, en passant par exemple par l'imidazolide intermédiaire obtenu par action du 1,1'-carbonylbis-1*H*-imidazole.

On peut, si on désire un composé final de formule générale (I) dans laquelle R₁ représente l'hydrogène, transformer un composé de formule générale (IV) dans laquelle R₁ représente l'hydrogène en un composé de formule générale (IV) dans laquelle R₁ représente un groupe protecteur tel que le groupe méthoxyméthyle, par exemple, par une réaction d'alkylation connue de l'homme du métier. On poursuit les transformations selon le schéma 1 jusqu'à l'obtention du composé de formule générale (IX), puis une hydrolyse acide de ce dernier, qui élimine en même temps le groupe protecteur méthoxyméthyle, aboutit à un composé de formule générale (X) dans laquelle R₁ représente un atome d'hydrogène.

Selon le schéma 2, on transforme le composé de formule générale (II), tel que défini ci-dessus, en un composé de formule générale (VI) par toute méthode connue de l'homme du métier, par exemple par une réaction électrophile en milieu acide. On traite ensuite ce dernier dans l'acide acétique, d'abord à température ambiante puis à la température de reflux, avec une phénylhydrazine éventuellement substituée par un groupe Y tel que défini ci-dessus. On obtient un composé de formule générale (VII), qu'on transforme en dérivé halogéné correspondant, de formule générale (VIII), par une réaction de type radicalaire, par exemple en utilisant le N-bromosuccinimide dans un solvant tel que le tétrachlorure de carbone, en présence d'un agent tel que le 2,2'-azobis(2-méthylpropionitrile). On traite ensuite le composé de formule générale (VIII) comme décrit à propos du schéma 1.

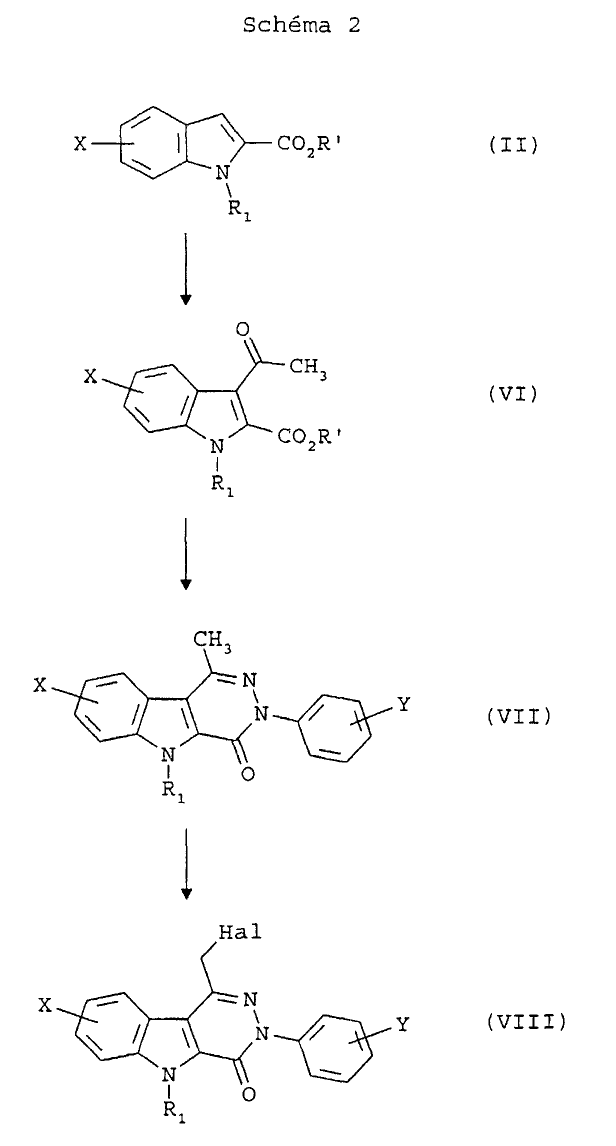

On peut, si on le désire, transformer un composé de formule générale (I) dans laquelle X représente un atome d'halogène en un composé de formule générale (I) dans laquelle X représente un groupe méthyle par toute réaction de couplage connue de l'homme du métier, par exemple en utilisant le tétraméthylétain en présence d'un complexe palladié.

On peut encore, si on le désire, transformer un composé de formule générale (I) dans laquelle X représente un atome de chlore en un composé de formule générale (I) dans laquelle R représente l'hydrogène, par exemple, par hydrogénation en présence de palladium sur charbon.

De même, on peut transformer un composé de formule générale (I), dans laquelle Y représente un groupe méthoxy, en composé dans la formule duquel Y représente un groupe hydroxy, par toute méthode connue, par exemple par action du tribromure de bore, dans un solvant chloré tel que le dichlorométhane.

Selon le schéma 3, on transforme un composé de formule générale (V), dans laquelle R₁ représente un groupe alkyle et X représente un atome de chlore, en un composé de formule générale (XI) par oxydation de la fonction alcool, par exemple en utilisant du dioxyde de manganèse dans un solvant tel que le dichlorométhane. On transforme ensuite l'aldéhyde (XI) en un nitrile de formule générale (XII), par action de 1-[(isocyanométhyl)sulfonyl]-4-méthylbenzène ("TosMIC") dans un solvant tel que le 1,2-diméthoxyéthane, en présence d'une base comme le 1,1-diméthyléthylate de potassium. On transforme ensuite le nitrile (XII) en un ester de formule générale (XIII), dans laquelle R" représente un groupe alkyle inférieur, par action d'un acide tel que le chlorure d'hydrogène, dans un solvant alcoolique de formule R"OH. Finalement on transforme cet ester (XIII) en amide secondaire ou tertiaire de formule générale (I) par action d'une amine de formule générale HNR₂R₃, dans laquelle R₂ et R₃ sont tels que définis ci-dessus, par exemple en présence d'un dérivé trialkylaluminium dans un solvant tel que du toluene.

Les composés de départ de formule générale (II) principalement lorsque R₁ représente l'hydrogène, sont décrits dans la littérature. Si on le désire on peut soumettre un composé dans la formule duquel R₁ représente l'hydrogène à une réaction d'alkylation pour aboutir à un composé dans la formule duquel R₁ représente un groupe alkyle. 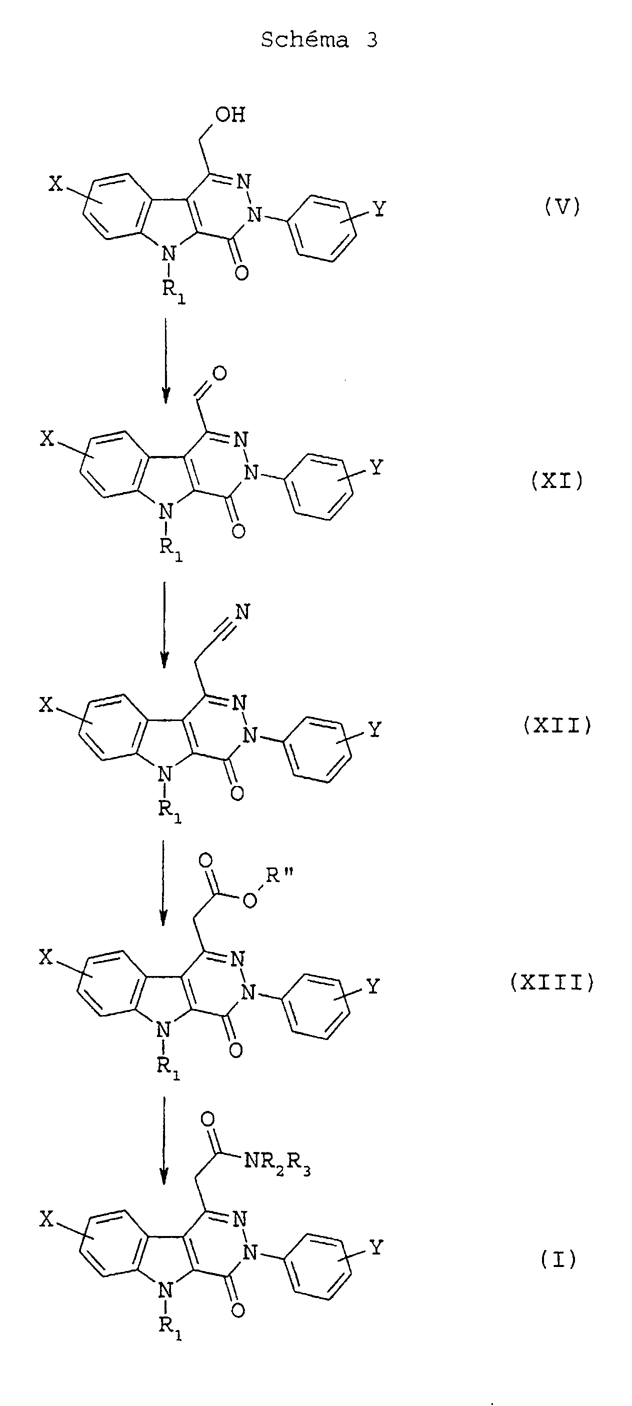 Les exemples qui vont suivre illustrent la préparation de quelques composés selon l'invention. Les microanalyses élémentaires, et les spectres I.R. et R.M.N. confirment les structures des composés obtenus.
Les numéros indiqués entre parenthèses dans les titres des exemples correspondent à ceux de la 1ère colonne du tableau 1 donné plus loin.
Dans les noms des composés, le tiret "-" fait partie du mot, et le tiret "-" ne sert que pour la coupure en fin de ligne ; il est à supprimer en l'absence de coupure, et ne doit être remplacé ni par un tiret normal ni par un espace.

### Exemple 1 (composé N°9)

5-Ethyl-8-fluoro-*N*,*N*-diméthyl-4-oxo-3-phényl-3,5-dihydro-4*H*-pyridazino[4,5-*b*]indole-1-acétamide.

### 1.1. 1-Ethyl-5-fluoro-1H-indole-2-carboxylate d'éthyle.

On agite pendant 2 h à température ambiante une suspension de 3,8 g (95 mmoles) d'hydrure de sodium à 60% (préalablement lavé à l'éther de pétrole) et de 15 g (72,4 mmoles) de 5-fluoro-*1H*-indole-2-carboxylate d'éthyle dans 100 ml de diméthylformamide. On ajoute ensuite 7,5 ml (93,7 mmoles) de iodoéthane en solution dans 20 ml de diméthylformamide. Après 10 h d'agitation à température ambiante, on verse le mélange réactionnel sur de l'eau glacée. On l'extrait par de l'éther diéthylique. On lave la phase organique plusieurs fois à l'eau, on la sèche sur sulfate de magnésium et on la concentre sous pression réduite. On obtient 17 g (72 mmoles) d'une huile jaune que l'on utilise telle quelle dans l'étape suivante.

### 1.2. 2-(Ethoxycarbonyl)-1-éthyl-5-fluoro-α-oxo-1H-indole-3-acétate de méthyle.

On chauffe au reflux pendant 6 h une solution de 17 g (72 mmoles) de 1-éthyl-5-fluoro-*1H*-indole-2-carboxylate d'éthyle et de 7,4 ml (84,5 mmoles) de chlorure d'oxalyle dans 500 ml de toluène. On ajoute 5 ml (57 mmoles) de chlorure d'oxalyle supplémentaire, on chauffe pendant 1 h à reflux et on laisse revenir à température ambiante. On ajoute 200 ml de méthanol, on agite pendant 10 min et on évapore les solvants sous pression réduite. On reprend l'huile résultante dans le dichlorométhane, on lave la phase organique à l'eau, on la sèche sur sulfate de magnésium et on la concentre sous pression réduite. Après recristallisation dans le propan-2-ol on obtient 14 g (43,6 mmoles) de composé sous la forme d'un solide blanc.

### 1.3. 5-Ethyl-8-fluoro-4-oxo-3-phényl-3,5-dihydro-4H-pyridazino[4,5-b]indole-1-carboxylate de méthyle.

A une solution de 14 g (43,6 mmoles) de 2-(éthoxycarbonyl)-1-éthyl-5-fluoro-α-oxo-*1H*-indole-3-acétate de méthyle dans 150 ml d'acide acétique on ajoute, à température ambiante, 18,4 ml (187,2 mmoles) de phénylhydrazine, on agite le mélange réactionnel pendant 30 min à température ambiante puis pendant 2 h au reflux.
On refroidit le milieu, on ajoute 100 ml d'eau, on sépare un insoluble par filtration en le lavant sur verre fritté avec un mélange 70/30 d'eau et d'acétone. On isole ainsi 10,5 g (28,6 mmoles) de solide blanc qu'on utilise tel quel dans l'étape suivante.

### 1.4. 5-Ethyl-8-fluoro-1-(hydroxyméthyl)-3-phényl-3,5-dihydro-4H-pyridazino[4,5-b]indol-4-one.

A une solution de 10 g (27,3 mmoles) de 5-éthyl-8-fluoro-4-oxo-3-phényl-3,5-dihydro-4*H*-pyridazino[4,5-*b*]indole-1-carboxylate de méthyle dans 200 ml de tétrahydrofurane et 5,8 ml de méthanol on ajoute, en plusieurs portions et à température ambiante, 5,1 g (135 mmoles) de borohydrure de sodium et on agite le mélange pendant 4 h au reflux.
On verse le mélange sur une solution glacée d'acide chlorhydrique 0,1 N, on sépare un insoluble par filtration en le lavant à l'eau et à l'éther diéthylique sur verre fritté, puis on le sèche. On isole 7,2 g (21,4 mmoles) de composé sous la forme d'un solide blanc qu'on utilise tel quel dans l'étape suivante.

### 1.5. 1-(Bromométhyl)-5-éthyl-8-fluoro-3-phényl-3,5-dihydro-4H-pyridazino[4,5-b]indol-4-one.

A une solution de 7,2 g (21,4 mmoles) de 5-éthyl-8-fluoro-1-(hydroxyméthyl)-3-phényl-3,5-dihydro-4*H*-pyridazino[4,5-*b*]indol-4-one et de 15,3 g (46,13 mmoles) de tétrabromure de carbone dans 500 ml de dichlorométhane on ajoute, en plusieurs portions, 11,5 g (43,84 mmoles) de triphénylphos_ phine et on agite la solution pendant 12 h à température ambiante.
On concentre au 1/3 le mélange sous pression réduite, on recueille le précipité par filtration, on le lave à l'éther et on le sèche sous pression réduite. On obtient 4 g (10 mmoles) de solide. On ajoute aux eaux-mères 300 ml de dichlo_ rométhane, 8 g (24,1 mmoles) de tétrabromure de carbone et 5 g (19,06 mmoles) de triphénylphosphine, on agite la solution pendant 12 h à température ambiante, puis on la concentre au 1/3. On recueille le précipité par filtration, on le lave à l'éther et on le sèche sous pression réduite. On obtient 3,2 g (8 mmoles) de solide supplémentaire.

### 1.6. 5-Ethyl-8-fluoro-4-oxo-3-phényl-3,5-dihydro-4H-pyridazino[4,5-b]indole-1-acétonitrile.

On agite vigoureusement pendant 12 h un mélange biphasique de 7,2 g (18 mmoles) de 1-(bromométhyl)-5-éthyl-8-fluoro-3-phényl-3,5-dihydro-4*H*-pyridazino[4,5-*b*]indol-4-one, de 3,53 g (72 mmoles) de cyanure de sodium et de 0,58 g (1,8 mmole) de bromure de tétrabutylammonium dans 300 ml de dichlorométhane et 150 ml d'eau.
On sépare la phase organique, on la lave plusieurs fois à l'eau, on la sèche sur sulfate de magnésium et on la concentre sous pression réduite. On isole 5,8 g (16,8 mmoles) de composé qu'on utilise tel quel dans l'étape suivante.

### 1.7. Acide 5-éthyl-8-fluoro-4-oxo-3-phényl-3,5-dihydro-4H-pyridazino[4,5-b]indole-1-acétique.

On chauffe pendant 2 h au reflux une solution de 5,8 g (16,8 mmoles) de 5-éthyl-8-fluoro-4-oxo-3-phényl-3,5-dihydro-4*H*-pyridazino[4,5-*b*]indole-1-acétonitrile dans 200 ml d'un mélange 1/1 d'acide chlorhydrique concentré et d'acide acétique glacial.
On refroidit la solution, on ajoute 100 ml d'eau, on recueille l'insoluble par filtration et on le lave abondamment à l'eau et à l'éther sur verre fritté.
On obtient, après séchage à l'étuve, 5,2 g (15 mmoles) d'un solide blanc qu'on utilise tel quel dans l'étape suivante.

### 1.8. 5-Ethyl-8-fluoro-N,N-diméthyl-4-oxo-3-phényl-3,5-dihydro-4H-pyridazino[4,5-b]indole-1-acétamide.

On agite une suspension de 1 g (2,73 mmoles) d'acide 5-éthyl-8-fluoro-4-oxo-3-phényl-3,5-dihydro-4*H*-pyridazino[4,5-*b*]indole-1-acétique et de 0,7 g (4,3 mmoles) de 1,1'-carbonylbis-1*H*-imidazole dans 200 ml de tétrahydrofurane pendant 3 h à 50°C.
On refroidit le mélange réactionnel à 25°C, on ajoute un excès de diméthylamine liquéfiée et on agite le mélange réactionnel pendant 12 h à température ambiante.
On le concentre sous pression réduite, on ajoute 100 ml de dichlorométhane et 100 ml d'eau, on sépare la phase organique, on la lave à l'eau, on la sèche sur sulfate de magnésium et on la concentre sous pression réduite. L'huile résultante cristallise dans l'éther.
Après filtration et recristallisation dans l'acétate d'éthyle on isole 0,75 g (1,9 mmoles) de cristaux blancs.
Point de fusion : 183-184°C.

### Exemple 2 (composé N°25).

1-[2-(8-Chloro-5-méthyl-4-oxo-3-phényl-3,5-dihydro-4*H*-pyridazino[4,5-*b*]indol-1-yl)-1-oxoéthyl]pyrrolidine.

### 2.1. 5-Chloro-2-(éthoxycarbonyl)-1-méthyl-α-oxo-1H-indole-3-acétate de méthyle.

On ajoute 15 ml (170 mmoles) de chlorure d'oxalyle à une solution de 31,5 g (133 mmoles) de 5-chloro-1-méthyl-*1H*-indole-2-carboxylate d'éthyle dans 100 ml de toluène chauffé à 60°C, et on chauffe à reflux pendant 1 h.
On refroidit la solution, on ajoute 50 ml de méthanol, on concentre sous pression réduite et on reprend le résidu par 100 ml de dichlorométhane, 50 ml d'eau, on ajoute de l'hydro génocarbonate de sodium, on sépare la phase organique, on la sèche sur sulfate de magnésium, on évapore les solvants sous pression réduite, on triture le résidu dans l'éther, on recueille le précipité par filtration et on le sèche sous pression réduite. On obtient 19 g (59 mmoles) de solide.
Point de fusion : 119-120°C.

### 2.2. 8-Chloro-5-méthyl-4-oxo-3-phényl-3,5-dihydro-4H-pyridazino[4,5-b]indole-1-carboxylate de méthyle.

On chauffe à reflux pendant 2 h une solution de 19 g (59 mmoles) de 5-chloro-2-(éthoxycarbonyl)-1-méthyl-α-oxo-*1H*-indole-3-acétate de méthyle et 26 g (240 mmoles) de phénylhydrazine dans 250 ml d'acide acétique. On refroidit le mélange, on ajoute 250 ml d'un mélange 1/1 d'eau et d'acétone et on laisse reposer le mélange à 4°C pendant 15 h.
On recueille le précipité par filtration, on le lave à l'eau et à l'acétone, on le sèche sous pression réduite.
On obtient 17,4 g (47 mmoles) de solide.
Point de fusion : 265-266°C.

### 2.3. 8-Chloro-1-(hydroxyméthyl)-5-méthyl-3-phényl-3,5-dihydro-4H-pyridazino [4,5-b]indol-4-one.

On chauffe à reflux pendant 4 h une solution de 17,2 g (47 mmoles) de 8-chloro-5-méthyl-4-oxo-3-phényl-3,5-dihydro-4*H*-pyridazino[4,5-*b*]indole-1-carboxylate de méthyle, de 8,7 g (230 mmoles) de borohydrure de sodium et de 9,4 ml (230 mmoles) de méthanol dans 300 ml de tétrahydrofurane. On refroidit le mélange, on le verse sur une solution agitée de 100 ml d'acide chlorhydrique 2N et de 100 ml de dichlorométhane. On recueille le précipité par filtration, on le lave à l'eau et au dichlorométhane et on le sèche sous pression réduite. On obtient 15 g (44 mmoles) de solide.
Point de fusion : 278-280°C.

### 2.4. 1-(Bromométhyl)-8-chloro-5-méthyl-3-phényl-3,5-dihydro-4H-pyridazino [4,5-b]indol-4-one.

On opère comme dans l'exemple 1.5 à partir de 15 g (44 mmoles) de 8-chloro-1-(hydroxyméthyl)-5-méthyl-3-phényl-3,5-dihydro-4*H*-pyridazino[4,5-*b*]indol-4-one. Après plusieurs traitement et purification par chromatographie sur gel de silice, on isole 15 g (37 mmoles) de solide.
Point de fusion : 253-254°C.

### 2.5. 8-Chloro-5-méthyl-4-oxo-3-phényl-3,5-dihydro-4H-pyridazino[4,5-b]indole-1-acétonitrile.

On opère comme dans l'exemple 1.6 à partir de 12,5 g (3 mmoles) de 1-(bromométhyl)-8-chloro-5-méthyl-3-phényl-3,5-dihydro-4*H*-pyridazino[4,5-*b*]indol-4-one dans un mélange de chloroforme et d'eau. Après purification sur colonne de gel de silice on obtient 10 g (28 mmoles) de solide.
Point de fusion : 230°C.

### 2.6. Acide 8-chloro-5-méthyl-4-oxo-3-phényl-3,5-dihydro-4H-pyridazino[4,5-b]indole-1-acétique.

On chauffe à 100°C pendant 4 h une solution de 10 g (28 mmoles) de 8-chloro-5-méthyl-4-oxo-3-phényl-3,5-dihydro-4*H*-pyridazino[4,5-*b*]indole-1-acétonitrile dans un mélange de 200 ml d'acide chlorhydrique concentré et de 200 ml d'acide acétique. On concentre sous pression réduite, on reprend le résidu par 250 ml d'eau et on recueille le précipité par filtration. On le lave à l'eau et on le sèche sous pression réduite. On obtient 10,2 g (27 mmoles) de solide.
Point de fusion : 206-208°C.

### 2.7. 1-[2-(8-Chloro-5-méthyl-4-oxo-3-phényl-3,5-dihydro-4H-pyridazino[4,5-b]indol-1-yl)-1-oxoéthyl]pyrrolidine.

On agite pendant 1 h à 50°C une solution de 1,5 g (4 mmoles) d'acide 8-chloro-5-méthyl-4-oxo-3-phényl-3,5-dihydro-4*H*-pyridazino[4,5-*b*]indole-1-acétique et de 1,2 g (7,4 mmoles) de 1,1'-carbonylbis-1*H*-imidazole dans le tétrahydrofurane. On refroidit le mélange et on ajoute un excès de pyrrolidine. Après 15 h d'agitation on recueille le précipité par filtration, on le lave à l'eau et à l'éther et on le recristallise dans le propan-2-ol. On obtient 0,65 g (1,5 mmoles) de solide.
Point de fusion : 261-262°C.

### Exemple 3 (composé N°27).

*N*,*N*,5-Triméthyl-4-oxo-3-phényl-3,5-dihydro-4*H*-pyridazino[4,5-*b*]indole-1-acétamide.

### 3.1. Acide 5-méthyl-4-oxo-3-phényl-3,5-dihydro-4H-pyridazino[4,5-b]indole-1-acétique.

On chauffe à reflux pendant 5 h une solution de 3,3 g (9 mmoles) d'acide 8-chloro-5-méthyl-4-oxo-3-phényl-3,5-dihydro-4*H*-pyridazino[4,5-*b*]indole-1-acétique, de 2,8 g (44 mmoles) de formiate d'ammonium et de 1,8 g de palladium sur charbon à 10% dans 500 ml d'éthanol.
On refroidit le mélange réactionnel, on ajoute du dichlorométhane, on élimine le catalyseur par filtration sur terre d'infusoires et on évapore le solvant sous pression réduite. On obtient 3 g (9 mmoles) de composé qu'on utilise tel quel dans l'étape suivante.

### 3.2. N,N,5-Triméthyl-4-oxo-3-phényl-3,5-dihydro-4H-pyridazino[4,5-b]indole-1-acétamide.

On agite une suspension de 1 g (3 mmoles) d'acide 5-méthyl-4-oxo-3-phényl-3,5-dihydro-4*H*-pyridazino[4,5-*b*]indole-1-acétique et de 0,7 g (4,3 mmoles) de 1,1'-carbonylbis-1*H*-imidazole dans 200 ml de tétrahydrofurane pendant 2 h à 60°C. On refroidit le mélange réactionnel à 25°C, on ajoute un excès de diméthylamine liquéfiée en solution dans le tétrahydrofurane et on agite le mélange réactionnel pendant 72 h à température ambiante.
On le concentre sous pression réduite, on ajoute 300 ml d'eau, on recueille le précipité par filtration, on le lave à l'eau et à l'éther et on le recristallise dans le propan-2-ol. On isole 0,75 g (2 mmoles) de solide.
Point de fusion : 214-215°C.

### Exemple 4 (composé N°26).

1-[2-(5-Méthyl-4-oxo-3-phényl-3,5-dihydro-4*H*-pyridazino[4,5-*b*]indol-1-yl)-1-oxoéthyl]pyrrolidine.

On opère comme dans l'exemple 3.2 à partir de 1 g (3 mmoles) d'acide 5-méthyl-4-oxo-3-phényl-3,5-dihydro-4*H*-pyridazino[4,5-*b*]indole-1-acétique et de la pyrrolidine en excès. On recristallise le produit dans le propan-2-ol. On obtient 0,5 g (1,3 mmoles) de solide.
Point de fusion : 214-215°C.

### Exemple 5 (composé N°31).

1-[2-(9-Bromo-5-méthyl-4-oxo-3-phényl-3,5-dihydro-4*H*-pyridazino[4,5-*b*]indol-1-yl)-1-oxoéthyl]pyrrolidine.

### 5.1. 2-Azido-3-(2-bromophényl)prop-2-énoate de méthyle.

On ajoute en 3 h, goutte à goutte, à une température de -10 à -8°C, sous azote et sous agitation mécanique, une solution de 75 ml (624 mmoles) de 2-bromobenzaldéhyde et de 252 g (2,2 moles) d'azidoacétate de méthyle dans 160 ml de méthanol à une solution de 476 ml de méthylate de sodium (30% dans le méthanol) dans 950 ml de méthanol. On maintient l'agitation pendant 2 h à une température en dessous de 5°C. On verse le mélange sur 1,5 kg de glace. On recueille le précipité par filtration, on le lave à l'eau et on le sèche sous pression réduite à l'abri de la lumière. On obtient 116 g (0,41 mole) de solide que l'on utilise rapidement dans l'étape suivante.

### 5.2. 4-Bromo-1H-indole-2-carboxylate de méthyle.

On ajoute, sous agitation mécanique, goutte à goutte en 4 h, une solution de 116 g (0,41 mole) de 2-azido-3-(2-bromophényl)prop-2-énoate de méthyle dans 1,5 l de toluène à une solution de 2 l de toluène chauffée à reflux et on maintient le reflux pendant 1 h supplémentaire. On évapore le solvant sous pression réduite et on reprend le résidu par 2 l de cyclohexane. On recueille le précipité par filtration, on le lave avec du toluène et on le sèche sous pression réduite. On isole 37,85 g (149 mmoles) de produit. On concentre les eaux-mères sous pression réduite et on purifie le résidu par chromatographie sur colonne de gel de silice.
On isole 12,2 g (48 mmoles) de produit supplémentaire.

### 5.3. 4-Bromo-1-méthyl-1H-indole-2-carboxylate de méthyle.

On opère comme dans l'exemple 1.1 à partir de 20 g (79 mmoles) de 4-bromo-1*H*-indole-2-carboxylate de méthyle, de 3,8 g d'hydrure de sodium à 60% et de 6 ml d'iodométhane. Après réaction, on évapore le solvant sous pression réduite et on reprend le résidu par de l'eau. On extrait le mélange par de l'acétate d'éthyle. On sèche la phase organique et on évapore le solvant sous pression réduite. On sèche le produit sous pression réduite. On obtient 20,6 g (77 mmoles) de solide.
Point de fusion : 85-86°C.

### 5.4. 3-Acétyl-4-bromo-1-méthyl-1H-indole-2-carboxylate de méthyle.

On ajoute 33 ml d'anhydride trifluoroacétique à une solution de 13,2 ml d'acide acétique, de 1,6 ml d'acide phosphorique et de 170 ml d'acétonitrile, on agite pendant 10 min à température ambiante et on ajoute une solution de 20,6 g (77 mmoles) de 4-bromo-1-méthyl-1*H*-indole-2-carboxylate de méthyle dans 120 ml d'acétonitrile. On agite le mélange pendant 4 h à température ambiante, on le verse sur de l'eau et on l'extrait par de l'éther. On sèche la phase organique sur sulfate de sodium, on évapore le solvant sous pression réduite, on reprend le résidu par un mélange de cyclohexane et de dichlorométhane. On recueille le précipité par filtration, on le lave à l'éther et on le sèche sous pression réduite. On obtient 18,8 g (61 mmoles) de produit.
On concentre les eaux-mères et on purifie le résidu par chromatographie sur colonne de gel de silice.
On isole 3,2 g (10 mmoles) de produit supplémentaire.
Point de fusion : 128°C.

### 5.5. 9-Bromo-1,5-diméthyl-3-phényl-3,5-dihydro-4H-pyridazino[4,5-b]indol-4-one.

On dissout à chaud 22 g (71 mmoles) de 3-acétyl-4-bromo-1-méthyl-1*H*-indole-2-carboxylate de méthyle dans 350 ml d'acide acétique. On ajoute 30 ml (300 mmoles) de phénylhydrazine, on agite pendant 1 h à température ambiante et on chauffe le milieu réactionnel à reflux pendant 5 h, à température ambiante pendant 15 h, puis de nouveau au reflux pendant 7 h.
On ajoute 28 ml de phénylhydrazine et on renouvelle le proccessus. On concentre sous pression réduite, on reprend le résidu par de l'eau, on recueille le précipité par filtration, on le lave à l'eau et on le sèche sous pression réduite. On le purifie par chromatographie sur colonne de gel de silice. On isole 11,1 g (30 mmoles) de produit.
Point de fusion : 189-190°C.

### 5.6. 9-Bromo-1-(bromométhyl)-5-méthyl-3-phényl-3,5-dihydro-4H-pyridazino[4,5-b]indol-4-one.

On chauffe à reflux pendant 5 h une solution de 10,1 g (27,1 mmoles) de 9-bromo-1,5-diméthyl-3-phényl-3,5-dihydro-4*H*-pyridazino[4,5-*b*]indol-4-one, de 6 g (34 mmoles) de *N*-bromo-succinimide et de 0,46 g (2,8 mmoles) de 2,2'-azobis(2-méthylpropionitrile). On rajoute 3 g de (17 mmoles) de N-bromosuccinimide et 0,23 g (1,4 mmoles) de 2,2'-azobis(2-méthylpropionitrile). On chauffe à reflux pendant 2 h, on abandonne à température ambiante pendant 15 h et on chauffe à nouveau à reflux pendant 5 h. On concentre le mélange sous pression réduite, on reprend le résidu à l'eau et on extrait par de l'acétate d'éthyle. On sèche la phase organique, on évapore le solvant sous pression réduite et on purifie le résidu par chromatographie sur colonne de gel de silice. On isole 5,9 g (13 mmoles) de produit.

### 5.7. 9-Bromo-5-méthyl-4-oxo-3-phényl-3,5-dihydro-4H-pyridazino[4,5-b]indole-1-acétonitrile.

On chauffe à reflux pendant 3 h sous agitation mécanique une solution de 6,4 g (14,3 mmoles) de 9-bromo-1-(bromométhyl)-5-méthyl-3-phényl-3,5-dihydro-4*H*-pyridazino[4,5-*b*]indol-4-one, de 3,6 g (73 mmoles) de cyanure de sodium et de 0,57 g (1 mmole) de bromure de tétrabutylammonium dans un mélange de 170 ml de dichlorométhane et de 85 ml d'eau. On décante et on extrait le milieu réactionnel au dichlorométhane. On lave la phase organique à l'eau, on la sèche sur sulfate de sodium et on évapore le solvant sous pression réduite. On obtient 5,6 g (14,2 mmoles) de produit que l'on utilise tel quel dans l'étape suivante.

### 5.8. Acide 9-bromo-5-méthyl-4-oxo-3-phényl-3,5-dihydro-4H-pyridazino[4,5-b]indole-1-acétique.

On chauffe à reflux pendant 6 h une solution de 4,8 g (12,2 mmoles) de 9-bromo-5-méthyl-4-oxo-3-phényl-3,5-dihydro-4*H*-pyridazino[4,5-*b*]indole-1-acétonitrile dans un mélange de 190 ml d'acide acétique et de 50 ml d'acide chlorhydrique concentré. On concentre sous pression réduite, on reprend par un mélange de dichlorométhane et d'eau. On alcalinise par de la soude à 30%, on décante et on extrait par du dichlorométhane. On acidifie la phase aqueuse par de l'acide chlorhydrique concentré en refroidissant par un bain de glaçe. On recueille le précipité par filtration, on le lave à l'eau et on le sèche sous pression réduite. On obtient 3,1 g (7,5 mmoles) de solide.

### 5.9. 1-[2-(9-Bromo-5-méthyl-4-oxo-3-phényl-3,5-dihydro-4H-pyridazino[4,5-b]indol-1-yl)-1-oxoéthyl]pyrrolidine.

On opère comme dans l'exemple 3.2 à partir de 3,1 g (7,5 mmoles) d'acide 9-bromo-5-méthyl-4-oxo-3-phényl-3,5-dihydro-4*H*-pyridazino[4,5-*b*]indole-1-acétique, de 1,4 g de 1,1'-carbonylbis-1*H*-imidazole et de 0,7 ml de pyrrolidine. Après réaction, on ajoute de l'eau, on recueille le précipité par filtration et on le sèche sous pression réduite. On le recristallise dans le propan-2-ol et on le lave par de l'éther et du pentane. On le sèche sous pression réduite.
On obtient 2,3 g (4,9 mmoles) de solide.
Point de fusion : 209-210°C.

### Exemple 6 (composé N°11).

9-Bromo-5-méthyl-*N*-méthyl-4-oxo-3-phényl-3,5-dihydro-4*H*-pyridazino[4,5-*b*]indole-1-acétamide.

On opère comme dans l'exemple 3.2 à partir de 0,78 g (1,9 mmoles) d'acide 9-bromo-5-méthyl-4-oxo-3-phényl-3,5-dihydro-4*H*-pyridazino[4,5-*b*]indole-1-acétique. On recristallise le produit dans le propan-2-ol.
On obtient 0,57 g (1,3 mmoles) de solide.
Point de fusion : 267-268°C.

### Exemple 7 (composé N°38).

1-[2-(5,9-diméthyl-4-oxo-3-phényl-3,5-dihydro-4*H*-pyridazino-[4,5-*b*] indol-1-yl)-1-oxoéthyl]pyrrolidine.

On chauffe à 120°C pendant 18 h dans un tube scellé une solution de 1,2 g (2,6 mmoles) de 1-[2-(9-bromo-5-méthyl-4-oxo-3-phényl-3,5-dihydro-4*H*-pyridazino[4,5-*b*] indol-1-yl)-1-oxoéthyl]pyrrolidine, de 0,22 g (0,3 mmoles) de chlorure de bis(triphénylphosphine)palladium (II), de 0,41 g (1,55 mmoles) de triphénylphosphine et de 1,5 ml (10,3 mmoles) de tétraméthylétain dans 15 ml de diméthylformamide. On concentre sous pression réduite, on reprend le résidu par du dichlorométhane et une solution d'hydrogénocarbonate de sodium. On lave la phase organique par une solution de fluorure de potassium à 10%, on la sèche sur sulfate de sodium, on évapore le solvant sous pression réduite et on purifie le résidu par chromatographie sur colonne de gel de silice. On recristallise le produit dans le propan-2-ol, on le lave à l'éther et au pentane et on le sèche sous pression réduite.
On obtient 0,82 g (2 mmoles) de solide.
Point de fusion : 214-215°C.

### Exemple 8 (composé N°23).

1-[2-(5-Méthyl-4-oxo-3-phényl-8-(phénylméthoxy)-3,5-dihydro-4*H*-pyridazino[4,5-*b*] indol-1-yl)-1-oxoéthyl]pyrrolidine.

### 8.1. Acide 5-méthyl-4-oxo-3-phényl-8-(phénylméthoxy)-3,5-dihydro-4H-pyridazino[4,5-b]indole-1-acétique.

On chauffe à reflux une solution de 1,15 g (2,7 mmoles) de 5-méthyl-4-oxo-3-phényl-8-(phénylméthoxy)-3,5-dihydro-4*H*-pyridazino[4,5-*b*]indole-1-acétonitrile et de 1,54 g (39 mmoles) d'hydroxyde de potassium dans un mélange de 10 ml d'eau et de 20 ml de 2-méthoxyéthanol. On extrait le milieu par du dichlorométhane, on acidifie la phase aqueuse, on l'extrait par de l'acétate d'éthyle, on sèche la phase organique sur sulfate de sodium et on évapore le solvant sous pression réduite. On isole 0,38 g (0,86 mmole) de solide que l'on utilise tel quel dans l'étape suivante.

### 8.2. 1-[2-(5-Méthyl-4-oxo-3-phényl-8-(phénylméthoxy)-3,5-dihydro-4H-pyridazino[4,5-b]indol-1-yl)-1-oxoéthyl]pyrrolidine

On opère comme dans l'exemple 3.2 à partir de 0,38 g (0,86 mmole) d'acide 5-méthyl-4-oxo-3-phényl-8-(phénylméthoxy)-3,5-dihydro-4*H*-pyridazino[4,5-*b*]indole-1-acétique. On obtient 0,35 g (0,7 mmole) de solide.
Point de fusion : 203-204°C.

### Exemple 9 (composé N°17)

*N*,*N*,5,8-tétraméthyl-4-oxo-3-phényl-3,5-dihydro-4*H*-pyridazino[4,5-*b*]indole-1-acétamide.

### 9.1. 1-(Chlorométhyl)-5,8-diméthyl-3-phényl-3,5-dihydro-4H-pyridazino [4,5-b] indol-4-one.

On ajoute 50 ml de pyridine à une suspension de 9,5 g (29,7 mmoles) de 1-(hydroxyméthyl)-5,8-diméthyl-3-phényl-3,5-dihydro-4*H*-pyridazino[4,5-*b*]indol-4-one dans 600 ml de tétrahydrofurane chauffée à 60°C. On ajoute 4,5 ml (59,4 mmoles) de chlorure de méthanesulfonyle et on laisse reposer le mélange pendant 15 h à température ambiante. On ajoute 500 ml de dichlorométhane, on élimine le précipité par filtration, on décante et on lave la phase organique à l'eau.
On la sèche sur sulfate de sodium et on évapore le solvant sous pression réduite. On obtient 5 g (14,8 mmoles) de produit que l'on utilise tel quel dans l'étape suivante.

### 9.2. 5,8-Diméthyl-4-oxo-3-phényl-3,5-dihydro-4H-pyridazino[4,5-b]indole-1-acétonitrile.

On agite pendant 2 h à 50°C une solution de 5 g (14,8 mmoles) de 1-(chlorométhyl)-5,8-diméthyl-3-phényl-3,5-dihydro-4*H*-pyridazino[4,5-*b*]indol-4-one, de 2,7 g (55 mmoles) de cyanure de sodium et de 0,5 g (3 mmoles) d'iodure de sodium dans un mélange de 50 ml de diméthylformamide et de 30 ml d'eau. On laisse reposer à température ambiante et on recueille le précipité par filtration. On le lave à l'eau et au pentane et on le sèche sous pression réduite.
On obtient 3 g (9,1 mmoles) de produit que l'on utilise tel quel dans l'étape suivante.

### 9.3. Acide 5,8-diméthyl-4-oxo-3-phényl-3,5-dihydro-4H-pyridazino[4,5-b]indole-1-acétique.

On opère comme dans l'exemple 1.7 à partir de 3,4 g (10,4 mmoles) de 5,8-diméthyl-4-oxo-3-phényl-3,5-dihydro-4*H*-pyridazino[4,5-*b*]indole-1-acétonitrile. On obtient 2 g (5,8 mmoles) de produit que l'on utilise tel quel dans l'étape suivante.

### 9.4. N,N,5,8-Tétraméthyl-4-oxo-3-phényl-3,5-dihydro-4H-pyridazino[4,5-b]indole-1-acétamide.

On opère comme dans l'exemple 3.2 à partir de 1 g (2,9 mmoles) d'acide 5,8-diméthyl-4-oxo-3-phényl-3,5-dihydro-4*H*-pyridazino[4,5-*b*]indole-1-acétique. On recristallise le produit dans le propan-2-ol. On obtient 0,5 g (1,3 mmole) de solide.
Point de fusion : 209-210°C.

### Exemple 10 (composé N°43).

8-Fluoro-*N*,*N*-diméthyl-4-oxo-3-phényl-3,5-dihydro-4*H*-pyridazino[4,5-*b*]indole-1-acétamide.

### 10.1. 2-(Ethoxycarbonyl)-5-fluoro-α-oxo-1H-indole-3-acétate d'éthyle.

On agite une solution de 40 ml (313 mmoles) de chloroglyoxylate d'éthyle et de 36 ml (313 mmoles) de tétrachlorure de titane dans 1 l de dichlorométhane pendant 15 min à température ambiante. On ajoute une solution de 50 g (241 mmoles) de 5-fluoro-*1H*-indole-2-carboxylate d'éthyle dans le dichlorométhane et on agite pendant 2 h à température ambiante. On verse le mélange sur de l'eau, on décante la phase organique, on la lave par de la soude diluée et on la sèche sur sulfate de sodium. On obtient 31,5 g (102 mmoles) de produit que l'on utilise tel quel dans l'étape suivante.

### 10.2. 8-Fluoro-4-oxo-3-phényl-3,5-dihydro-4H-pyridazino[4,5-b]indole-1-carboxylate d'éthyle.

On opère comme dans l'exemple 1.3 à partir de 5 g (16,2 mmoles) de 2-(éthoxycarbonyl)-5-fluoro-α-oxo-*1H*-indole-3-acétate d'éthyle et de 9,5 ml de phénylhydrazine dans 150 ml d'acide acétique. On isole 3,9 g (11 mmoles) de solide que l'on utilise tel quel dans l'étape suivante.

### 10.3. 8-Fluoro-5-(méthoxyméthyl)-4-oxo-3-phényl-3,5-dihydro-4H-pyridazino[4,5-b]indole-1-carboxylate d'éthyle.

On ajoute une suspension de 3,9 g (11 mmoles) de 8-fluoro-4-oxo-3-phényl-3,5-dihydro-4*H*-pyridazino[4,5-*b*]indole-1-carboxylate d'éthyle dans 200 ml de diméthylformamide à une suspension de 0,66 g (16 mmoles) d'hydrure de sodium dans 100 ml de diméthylformamide. On agite pendant 1,5 h à température ambiante et on ajoute une solution de 1,15 ml (14,3 mmoles) de chlorométhoxyméthane dans 10 ml de tétrahydrofurane. On agite pendant 2 h, on ajoute une solution d'acide chlorhydrique diluée, on recueille le précipité par filtration et on le sèche sous pression réduite. On isole 4,1 g (10,4 mmoles) de solide que l'on utilise tel quel dans l'étape suivante.

### 10.4. 8-Fluoro-1-(hydroxyméthyl)-5-(méthoxyméthyl)-3-phényl-3,5-dihydro-4H-pyridazino[4,5-b]indol-4-one.

On opère comme dans l'exemple 1.4 à partir de 4,1 g (10,4 mmoles) de 8-fluoro-5-(méthoxyméthyl)-4-oxo-3-phényl-3,5-dihydro-4*H*-pyridazino[4,5-*b*]indole-1-carboxylate d'éthyle. On isole 3,2 g (9 mmoles) de solide que l'on utilise tel quel dand l'étape suivante.

### 10.5. (1-Bromométhyl)-8-fluoro-5-(méthoxyméthyl)-3-phényl-3,5-dihydro-4H-pyridazino[4,5-b]indol-4-one.

On ajoute 4,5 g (17,1 mmoles) de triphénylphosphine à une solution de 3,2 g (9 mmoles) de 8-fluoro-1-(hydroxyméthyl)-5-méthoxyméthyl-3-phényl-3,5-dihydro-4*H*-pyridazino[4,5-*b*]indol-4-one et de 6,3 g (19 mmoles) de tétrabromométhane dans 200 ml de dichlorométhane. On agite pendant 2 h, on ajoute 50 ml de cyclohexane, on recueille le précipité par filtration et on le sèche sous pression réduite. On isole 2,9 g (7 mmoles) de solide que l'on utilise tel quel dans l'étape suivante.

### 10.6. 8-Fluoro-5-(méthoxyméthyl)-4-oxo-3-phényl-3,5-dihydro-4H-pyridazino[4,5-b]indole-1-acétonitrile.

On agite vigoureusement, pendant 12 h, un mélange biphasique de 2,8 g (6,7 mmoles) de 1-(bromométhyl)-8-fluoro-5-(méthoxyméthyl)-3-phényl-3,5-dihydro-4*H*-pyridazino[4,5-*b*]indol-4-one, de 1,3 g (26 mmoles) de cyanure de sodium et de 0,23 g (0,7 mmole) de bromure de tétrabutylammonium dans 100 ml de dichlorométhane et 100 ml d'eau. On sépare la phase organique, on la lave plusieurs fois à l'eau, on la sèche sur sulfate de magnésium et on la concentre sous pression réduite. On ajoute de l'éther diéthylique, on recueille le précipité par filtration et on le sèche sous pression réduite. On isole 2,1 g (5,8 mmoles) de solide que l'on utilise tel quel dans l'étape suivante.

### 10.7. Acide 8-fluoro-'-oxo-3-phényl-3,5-dihydro-4H-pyridazino[4,5-b]indole-1-acétique.

On opère comme dans l'exemple 1.7 à partir de 1,2 g (3,3 mmmoles) de 8-fluoro-5-méthoxyméthyl-4-oxo-3-phényl-3,5-dihydro-4*H*-pyridazino[4,5-*b*]indole-1-acétonitrile. On isole après filtration et séchage 1,1 g (3,2 mmoles) de solide qu'on utilise tel quel dans l'étape suivante.

### 10.8. 8-Fluoro-N,N-diméthyl-4-oxo-3-phényl-3,5-dihydro-4H-pyridazino [4,5-b]indole-1-acétamide.

On agite à 40°C pendant 3 h une suspension de 1,7 g (10,5 mmoles) de 1,1'-carbonylbis-1*H*-imidazole et de 3 g (8,9 mmoles) d'acide 8-fluoro-3-phényl-3,5-dihydro-4*H*-pyridazino-[4,5-*b*]indole-1-acétique dans 300 ml de tétrahydrofurane. On refroidit à température ambiante, on ajoute 10 ml de diméthylamine liquéfiée et on agite pendant 2 h. Après une nuit de repos, on ajoute 300 ml d'eau, on recueille le précipité par filtration et on le recristallise dans le diméthylformamide. On obtient 0,8 g (2,2 mmoles) de solide. Point de fusion : 289-290°C.

### Exemple 11 (composé N°52).

7-Chloro-*N*,*N*-diéthyl-5-méthyl-4-oxo-3-phenyl-3,5-dihydro-4*H*-pyridazino[4,5-*b*]indole-1-acetamide.

### 11.1. 6-Chloro-1-méthyl-1H-indole-2-carboxylate d'éthyle.

On opère comme dans l'exemple 1.1 à partir de 8.0 g (35.8 mmoles) de 6-chloro-1*H*-indole-2-carboxylate d'éthyle, de 1,8 g d'hydrure de sodium à 60% et de 2,8 ml d'iodométhane. Après réaction, on évapore le solvant sous pression réduite et on reprend le résidu par de l'eau. On extrait le mélange par du dichlorométhane, on sèche la phase organique, on filtre et on évapore le solvant sous pression réduite. On purifie le résidu par chromatographie sur colonne de gel de silice.
On isole 8,5 g (35,8 mmoles) d'un composé blanc cristallin.
Point de fusion : 75,5-76,5°C.

### 11.2. 6-Chloro-2-(éthoxycarbonyl)-1-méthyl-α-oxo-1H-indole-3-acétate d'éthyle.

A une solution de 4 ml (36 mmoles) de chlorooxoacétate d'éthyle dans 100 ml de 1,2-dichloroéthane, on ajoute 4 ml (36,4 mmoles) de chlorure de titane IV. On agite le mélange réactionnel pendant 30 min à température ambiante, puis on ajoute 7,8 g (32,8 mmoles) de 6-chloro-1-méthyl-1*H*-indole-2-carboxylate d'ethyle et on agite le mélange réactionnel pendant 4 heures à température ambiante.
On refroidit le milieu, on ajoute 200 ml de dichlorométhane et 100 ml d'eau. On décante la phase organique, on la lave à l'eau, on la sèche sur sulfate de sodium, on la filtre, on la concentre sous pression réduite et on purifie le résidu par chromatographie sur colonne de gel de silice.
On isole 9,4 g (27,7 mmoles) de produit.
Point de fusion : 94-95°C.

### 11.3. 7-Chloro-5-méthyl-4-oxo-3-phényl-3,5-dihydro-4H-pyridazino[4,5-b]indole-1-carboxylate d'éthyle.

A une solution de 4,6 g (13,6 mmoles) de 6-Chloro-2-(éthoxycarbonyl)-1-méthyl-α-oxo-1*H*-indole-3-acétate d'éthyle dans 120 ml d'acide acétique, on ajoute à température ambiante 4 ml (40,6 mmoles) de phénylhydrazine. On agite le mélange réactionnel pendant 30 min à température ambiante, puis pendant 4 heures au reflux.
On refroidit le milieu, on ajoute 350 ml de dichlorométhane et 100 ml d'eau. On décante la phase organique, on la lave avec une solution aqueuse saturée d'hydrogénocarbonate de sodium, puis avec de l'eau, on la sèche sur sulfate de sodium, on la filtre, on la concentre sous pression réduite et on purifie le résidu par chromatographie sur colonne de gel de silice.
On isole 4,1 g (10,7 mmoles) de produit.
Point de fusion : 216-218,5°C.

### 11.4. 7-Chloro-1-(hydroxyméthyl)-5-méthyl-3-phényl-3,5-dihydro-4H-pyridazino[4,5-b]indol-4-one.

A une solution de 4,04 g (10,6 mmoles) de 7-chloro-5-méthyl-4-oxo-3-phényl-3,5-dihydro-4*H*-pyridazino[4,5-*b*]indole-1-carboxylate d'ethyle dans 150 ml de tétrahydrofuranne, on ajoute 2,5 g (66,1 mmoles) de borohydrure de sodium. Sous agitation, on ajoute progressivement 2,25 ml de méthanol, puis on chauffe le mélange au reflux durant 5 heures.
On verse le mélange sur une solution glacée d'acide chlorhydrique 1 M, on sépare un insoluble par filtration sur verre fritté, que l'on lave à l'eau et à l'éther diéthylique, puis on le sèche.
On isole 3,3 g (9,7 mmoles) de composé sous forme d'un solide blanc qu'on utilise tel quel dans l'étape suivante.
Point de fusion : 219-220,5°C.

### 11.5. 7-chloro-5-méthyl-4-oxo-3-phényl-3,5-dihydro-4H-pyridazino[4,5-b]indole-1-carboxaldéhyde.

A une solution de 3,3 g (9,7 mmoles) de 7-chloro-1-(hydroxyméthyl)-5-méthyl-3-phényl-3,5-dihydro-4*H*-pyridazino[4,5-*b*]-indol-4-one dans 300 ml de dichlorométhane, on ajoute 5,7 g (65,6 mmoles) de dioxyde de manganèse et on agite le mélange réactionnel pendant 24 heures à reflux.
On refroidit le milieu, on le filtre sur membrane Téflon™, on rince le solide avec du dichlorométhane, puis on concentre le filtrat sous pression réduite.
On isole 2,88 g (8,53 mmoles) de composé sous forme d'un solide blanc qu'on utilise tel quel dans l'étape suivante. Point de fusion : 235-236°C.

### 11.6. 7-Chloro-5-méthyl-4-oxo-3-phényl-3,5-dihydro-4H-pyridazino[4,5-b]indole-1-acétonitrile.

A une solution de 2,14 g (10,96 mmol) de 1-[(isocyanométhyl)sulfonyl]-4-méthylbenzène dans 50 ml de 1,2-diméthoxyéthane, on ajoute, par petites portions, 1,27 g (10,96 mmoles) de 1,1-diméthyléthylate de potassium, on agite le mélange réactionnel pendant 30 min à -60°C, on ajoute 2,88 g (8,53 mmoles) de 7-chloro-5-méthyl-4-oxo-3-phényl-3,5-dihydro-4*H*-pyridazino[4,5-*b*]indole-1-carboxaldéhyde et on agite le mélange réactionnel durant 3 heures 30 min à -60°C. On ajoute 9 ml de méthanol et on agite encore le mélange réactionnel 30 min à température ambiante et 1 heure à reflux.
On refroidit le milieu, on le concentre sous pression réduite, on ajoute au résidu de l'eau, 5 ml d'acide acétique et 200 ml de dichlorométhane, on décante la phase organique et on extrait la phase aqueuse avec du dichlorométhane. On réunit les phases organiques, on les lave à l'eau, on les sèche sur sulfate de sodium, on les filtre, on les concentre sous pression réduite et on purifie le résidu par chromatographie sur colonne de gel de silice.
On isole 1,87 g (5,36 mmoles) de composé sous forme d'un solide blanc qu'on utilise tel quel dans l'étape suivante.
Point de fusion : 305-315°C.

### 11.7. 7-Chloro-5-méthyl-4-oxo-3-phényl-3,5-dihydro-4H-pyridazino[4,5-b]indole-1-acétate de méthyle.

A une solution de 1,83 g (5,25 mmoles) de 7-chloro-5-méthyl-4-oxo-3-phényl-3,5-dihydro-4*H*-pyridazino[4,5-*b*]indole-1-acétonitrile dans 250ml de méthanol, on ajoute du chlorure d'hydrogène jusqu'à saturation de la solution et on agite le mélange réactionnel pendant 4 heures à reflux.
On refroidit le milieu, on concentre le mélange réactionnel sous pression réduite, on ajoute au résidu 25 ml d'eau et 25 ml de méthanol. Après agitation on recueille l'insoluble par filtration, on le lave à l'eau et à l'éther diéthylique, on le sèche et on le purifie par chromatographie sur colonne de gel de silice.
On isole 1,00 g (2,62 mmoles) de composé sous forme d'un solide blanc.
Point de fusion : 188,5-190°C.

### 11.8. 7-Chloro-N,N-diéthyl-5-méthyl-4-oxo-3-phényl-3,5-dihydro-4H-pyridazino[4,5-b]indole-1-acétamide.

Sous argon, à une solution de 2 ml (4 mmoles) de triméthylaluminium (2M dans le toluène) dans 30 ml de toluène on ajoute, à 0°C, 0,41 ml (4 mmoles) de diéthylamine, on agite le mélange réactionnel pendant 20 min à température ambiante, on ajoute 0,095 g (0,25 mmoles) de 7-chloro-5-méthyl-4-oxo-3-phényl-3,5-dihydro-4*H*-pyridazino[4,5-*b*] indole-1-acétate de méthyle et on agite le mélange réactionnel pendant 4 h à reflux.
On refroidit le milieu à 4°C, on ajoute 3 ml d'eau et du dichlorométhane, on filtre la solution, et on concentre le filtrat sous pression réduite.
On ajoute au résidu de l'eau, de d'acide chlorhydrique 1M et 150 ml de dichlorométhane, on sépare la phase organique, on la lave à l'eau, on la sèche sur sulfate de sodium, on la filtre, on la concentre sous pression réduite et on purifie le résidu par chromatographie sur colonne de gel de silice.
Après recristallisation dans de l'éther diéthylique on isole 0,10 g (0,24 mmoles) de composé sous forme d'un solide blanc à l'aspect cotonneux.
Point de fusion : 167-168°C.

Le tableau qui suit illustre les structures chimiques et les propriétés physiques de quelques composés selon l'invention.

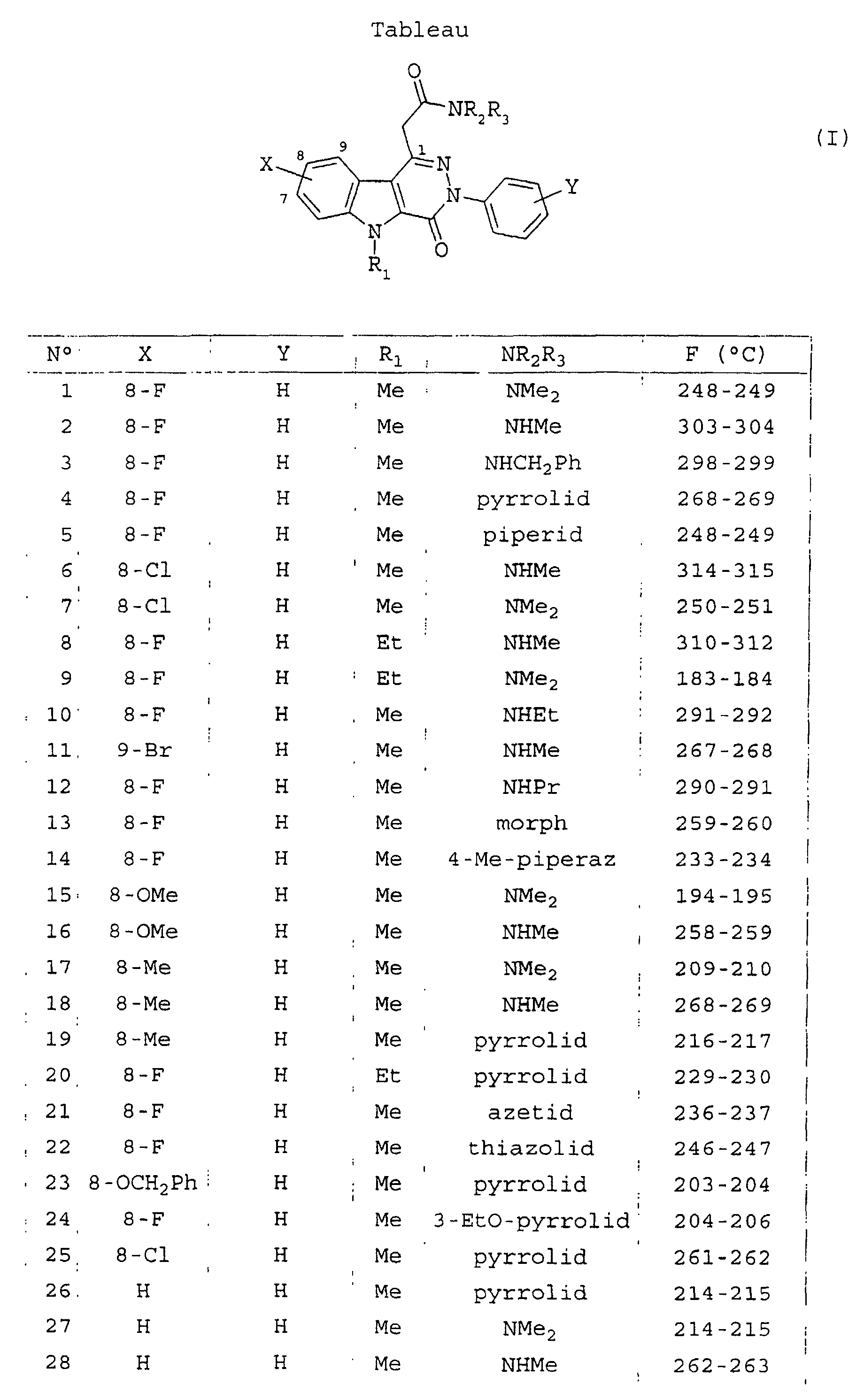

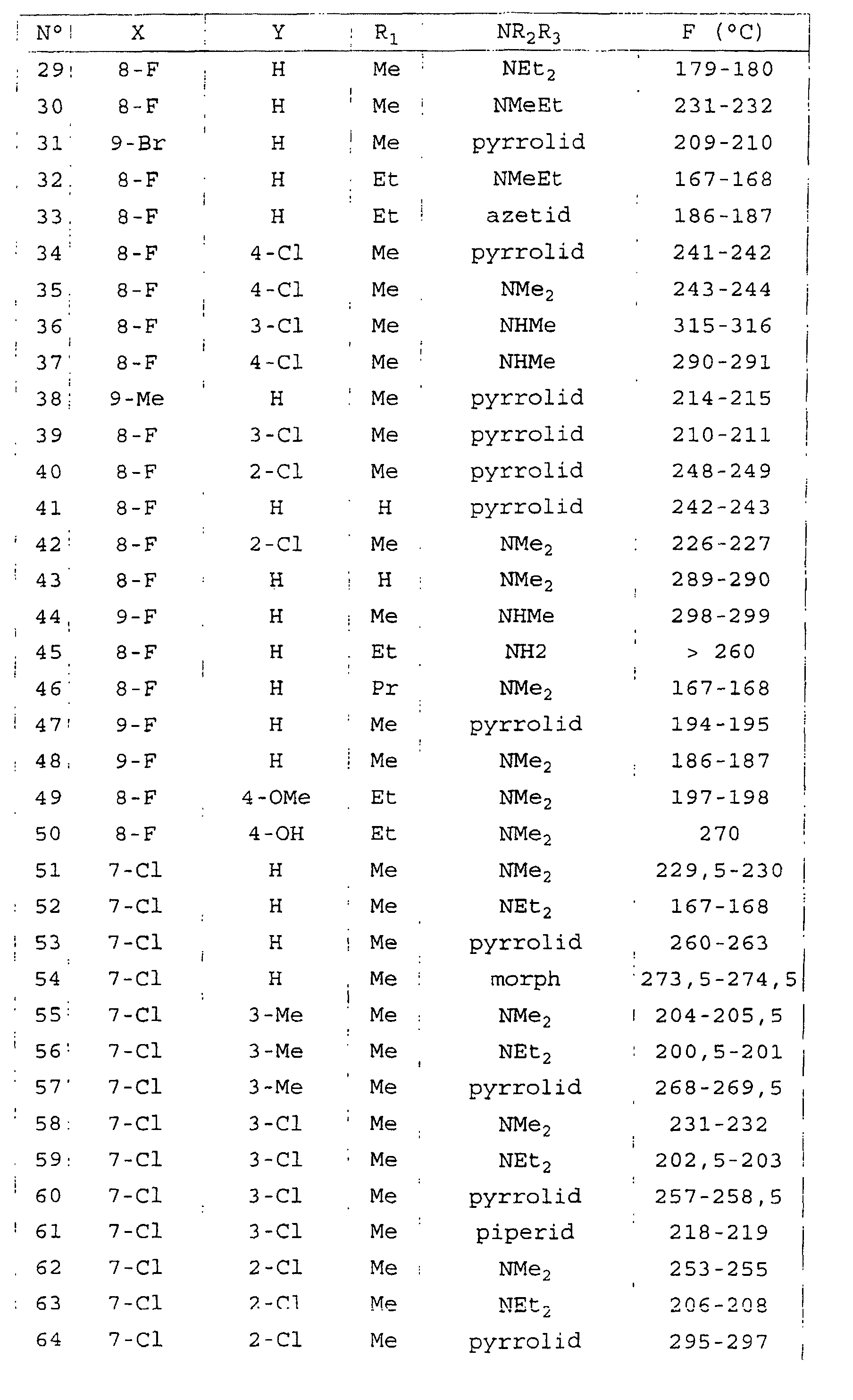

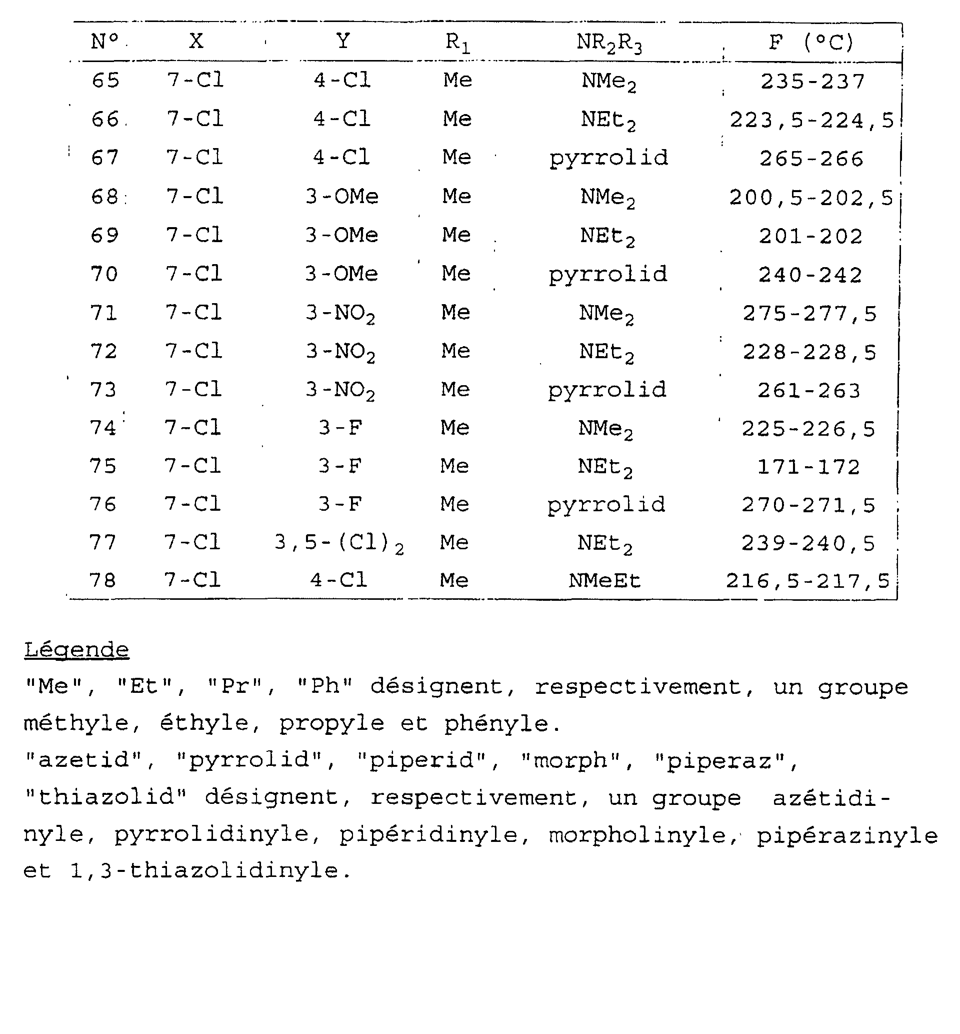

Les composés de l'invention ont été soumis à des essais pharmacologiques qui ont mis en évidence leur intérêt comme substances à activités thérapeutiques.

### Etude des liaisons membranaires vis-à-vis des récepteurs ω₁ du cervelet (benzodiazépiniques de type 1) et ω₂ de la moëlle épinière (benzodiazépiniques de type 2).

L'affinité des composés pour les récepteurs ω₁ du cervelet et ω₂ de la moëlle épinière a été déterminée selon une variante de la méthode décrite par S. Z. Langer et S. Arbilla dans *Fund. Clin. Pharmacol.,* **2,** 159-170 (1988), avec utilisation de ³H-flumazenil au lieu de ³H-diazepam comme radioligand.
On homogénéise le tissu du cervelet ou de la moëlle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du ³H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous pression réduite sur des filtres Whatman GF/B® et on les lave immédiatement avec du tampon glacé. La liaison spécifique du ³H-flumazenil est déterminée en présence de diazépam 1 µM non marqué. On analyse les données selon les méthodes usuelles et on calcule la concentration CI₅₀, concentration qui inhibe de 50% la liaison du ³H-flumazenil.
Les CI₅₀ des composés de l'invention les plus affins se situent entre 5 et 1000 nM pour les récepteurs ω₁ du cervelet, et entre 20 et 1000 nM pour les récepteurs ω₂ de la moëlle épinière.

### Etude de l'activité anxiolytique.

### Test de conflit de prise de boisson.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48h, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés les plus actifs se situent, dans cet essai, entre 0,1 et 10 mg/kg par la voie intrapéritonéale ou orale.

### Test du labyrinthe surélevé en croix.

Le protocole de cet essai est une modification de celui décrit par S. Pellow et S. File dans *Pharmacol. Biochem. Behav.* (1986) **24** 525-529.
Après une période d'habituation à la pièce expérimentale de 24 h environ, les rats sont placés individuellement sur la plate-forme centrale, le museau dirigé vers un des bras fermés, et observés pendant 4 min par l'intermédiaire d'une caméra vidéo. On enregistre le temps passé par l'animal dans les bras ouverts, le nombre d'entrées dans les bras fermés et dans les bras ouverts, le nombre de tentatives d'entrée dans les bras ouverts suivies par une réponse d'évitement et l'exploration des rebords dans les bras ouverts. Les résultats sont exprimés pour chaque animal : 1° en pourcentage de passages dans les bras ouverts par rapport au nombre total d'entrées dans les quatres bras de l'appareil, 2° en pourcentage de temps passé dans les bras ouverts par rapport à la durée totale du test (4 min), 3° en nombre total de tentatives avortées effectuées par l'animal, 4° en nombre total d'explorations.
Les produits à étudier sont administrés par voie intrapéritonéale ou orale à doses croissantes.
Les résultats sont exprimés par la dose efficace minimale (DEM) qui produit soit une augmentation significative (activité dans les bras ouverts) soit une diminution significative (tentatives) par rapport aux performances observées chez les animaux témoins.
Les DEM des composés les plus actifs se situent, dans cet essai, entre 0,1 et 20 mg/kg par la voie intrapéritonéale ou orale.

### Etude de l'activité hypnotique.

L'activité sédative ou hypnotique des composés a été déterminée par l'observation de leur action sur l'électrocorticogramme du rat, selon la méthode décrite par H. Depoortere, *Rev. E.E.G. Neurophysiol*., **10,** 3, 207-214 (1980) et par H. Depoortere et M. Decobert, *J. Pharmacol. (Paris)*, **14,** 2, 195-265 (1983).
Les produits à étudier ont été administrés par voie intrapéritonéale à doses croissantes. Les composés les plus actifs induisent des tracés de sommeil à des doses allant de 0,1 à 30 mg/kg.

### Etude de l'activité anticonvulsivante.

### Activité vis-à-vis des convulsions cloniques induites chez le rat par injection de pentétrazol.

Le protocole de cet essai est une modification de celui décrit par E. A. Swinyard et J. H. Woodhead dans *Antiepileptic Drugs,* Raven Press, New York, 111-126 (1982). Les produits à tester sont administrés aux animaux par voie intrapéritonéale 30 min avant une injection intraveineuse de 20 mg/kg de pentétrazol. Immédiatement après l'injection on note pendant 5 min le nombre d'animaux présentant des convulsions cloniques.
Les résultats sont exprimés par la DA₅₀, dose qui protège 50% des animaux, calculée selon la méthode de J. T. Lichtfield et F. Wilcoxon (*J. Pharm. Exp. Ther.* (1949) **96** 99-113) à partir de 3 ou 4 doses administrées chacune à un groupe de 8 à 10 rats.
Les DA₅₀ des composés les plus actifs se situent entre 0,1 et 10 mg/kg par la voie intrapéritonéale ou orale.

### Etude de l'activité anticonvulsivante.

### Activité vis-à-vis des convulsions induites chez la souris par l'isoniazide

L'activité intrinsèque des composés est déterminée par le temps de latence d'apparition des convulsions induites par l'administration sous-cutanée d'isoniazide (800 mg/kg) simultanément avec le composé à tester, injecté par voie intrapéritonéale, selon le protocole décrit par G. Perrault, E. Morel, D. Sanger et B. Zivkovic dans *Eur. J. Pharmacol*., **156,** 189-196 (1988). Les résultats sont exprimés par la DA₅₀, dose qui produit 50% de l'effet maximal, par rapport aux animaux témoins, déterminée à partir de 3 ou 4 doses administrées chacune à un groupe de 8 à 10 souris.
Les DA₅₀ des composés les plus actifs se situent, dans cet essai, entre 1 et 20 mg/kg par la voie intrapéritonéale et, selon les composés, l'effet maximal peut aller jusqu'à 400%.

Les résultats des essais effectués sur les composés de l'invention montrent que, *in vitro,* ils déplacent le ³H-flumazénil de ses sites de liaison spécifiques ω₁ au niveau du cervelet et ω₂ de la moëlle épinière ; ils présentent une affinité pour les sites ω₁ et ω₂ situés au sein du complexe macromoléculaire GABA_{A}-sites ω-canal chlorure.
*In vivo* ils se comportent comme des agonistes, complets ou partiels, vis-à-vis de ces sous-types de récepteurs.
Ils possèdent des propriétés anxiolytiques, hypnotiques et anticonvulsivantes.
Par conséquent ils peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, du tabac, des stupéfiants, etc.
Ils peuvent également être utilisés pour le traitement de la maladie de Parkinson ainsi que de tous types de syndromes extrapyramidaux.
Enfin, ils peuvent être utilisés dans la prémédication et comme anesthésiques généraux pour l'induction et/ou le maintien de l'anesthésie, ou comme anesthésiques locaux, éventuellement associés à d'autres anesthésiques et/ou des myorelaxants et/ou des analgésiques.

Ils peuvent être présentés sous toutes formes de compositions appropriées à l'administration entérale, parentérale ou transdermique, telles que comprimés, dragées, gélules, capsules, suspensions ou solutions buvables ou injectables telles que sirops ou ampoules, timbres transdermiques ("patch"), etc, associés à des excipients convenables, et dosés pour permettre une administration journalière de 1 à 1000 mg de substance active.

## Revendications

1. Composé répondant à la formule générale (I) 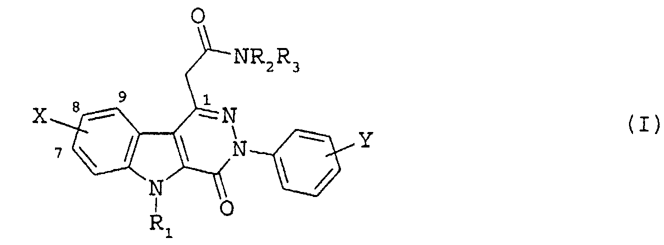 dans laquelle
X représente un atome d'hydrogène ou d'halogène ou un groupe méthyle, méthoxy ou phénylméthoxy,
Y représente un atome d'hydrogène, un ou deux atomes d'halogène ou un groupe méthyle, hydroxy, méthoxy ou nitro,
R₁ représente un atome d'hydrogène ou un groupe (C₁-C₄)alkyle,
R₂ et R₃ représentent chacun, indépendamment l'un de l'autre, un atome d'hydrogène, un groupe (C₁-C₄)alkyle ou un groupe phénylméthyle,
ou bien R₂ et R₃ forment, avec l'atome d'azote qui les porte, un groupe azétidinyle, pyrrolidinyle, 3-éthoxypyrrolidinyle, pipéridinyle, morpholinyle, 4-méthylpipérazinyle ou 1,3-thiazolidinyle.

2. Composé selon la revendication 1, **caractérisé en ce que** X est en position 8 ou 9 et représente un atome d'hydrogène ou d'halogène, Y représente un atome d'hydrogène, R₁ représente un groupe méthyle ou éthyle, R₂ représente un atome d'hydrogène ou un groupe méthyle, R₃ représente un groupe méthyle, ou bien R₂ et R₃ forment, avec l'atome d'azote qui les porte, un cycle azétidinyle ou pyrrolidinyle.

3. Médicament **caractérisé en ce qu'**il consiste en un composé selon l'une des revendications 1 et 2.

4. Composition pharmaceutique **caractérisée en ce qu'**elle contient un composé selon l'une des revendications 1 et 2, associé à un excipient.

## Patentansprüche

1. Verbindung der allgemeinen Formel (I) 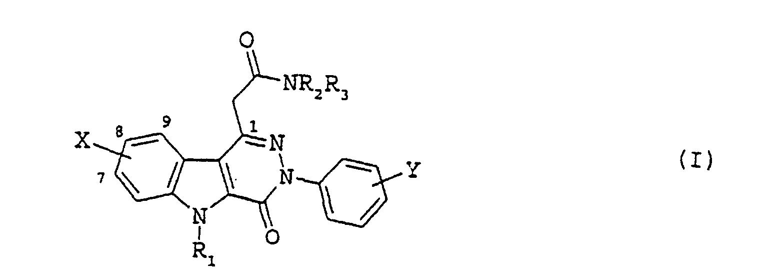 in der
X ein Wasserstoffatom, ein Halogenatom, eine Methyl-, Methoxy- oder Phenylmethoxygruppe,
Y ein Wasserstoffatom, ein oder zwei Halogenatome oder eine Methyl-, Hydroxy-, Methoxy- oder Nitrogruppe,
R₁ ein Wasserstoffatom oder eine (C₁-C₄)-Alkylgruppe,
R₂ und R₃ jeweils unabhängig voneinander ein Wasserstoffatom, eine (C₁-C₄)-Alkylgruppe oder eine Phenylmethylgruppe,
oder R₂ und R₃ gemeinsam mit dem sie tragenden Stickstoffatom eine Azetidinyl-, Pyrrolidinyl-, 3-Ethoxypyrrolidinyl-, Piperidinyl-, Morpholinyl-, 4-Methylpiperazinyl- oder 1,3-Thiazolidinylgruppe bedeuten.

2. Verbindung nach Anspruch 1, **dadurch gekennzeichnet, daß** X in der Stellung 8 oder 9 steht und ein Wasserstoffatom oder ein Halogenatom bedeutet, Y ein Wassestoffatom darstellt, R₁ eine Methylgruppe oder eine Ethylgruppe bedeutet, R₂ ein Wasserstoffatom oder eine Methylgruppe bedeutet, R₃ eine Methylgruppe bedeutet oder R₂ und R₃ zusammen mit dem sie tragenden Stickstoffatom einen Azetidinyl- oder Pyrrolidinylring bilden.

3. Arzneimittel, **dadurch gekennzeichnet, daß** es aus einer Verbindung nach einem der Ansprüche 1 und 2 besteht.

4. Pharmazeutische Zubereitung, **dadurch gekennzeichnet, daß** sie eine Verbindung nach einem der Ansprüche 1 und 2 zusammen mit einem Trägermaterial enthält.

## Claims

1. Compound corresponding to the general formula (I) 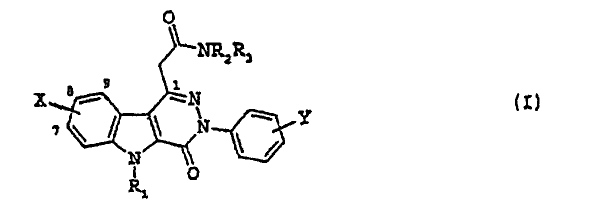 in which
X represents a hydrogen or halogen atom or a methyl, methoxy or phenylmethoxy group,
Y represents a hydrogen atom, one or two halogen atoms or a methyl, hydroxyl, methoxy or nitro group,
R₁ represents a hydrogen atom or a (C₁-C₄)alkyl group,
R₂ and R₃ each represent, independently of one another, a hydrogen atom, a (C₁-C₄)alkyl group or a phenylmethyl group,
or else R₂ and R₃ form, with the nitrogen atom which carries them, an azetidinyl, pyrrolidinyl, 3-ethoxypyrrolidinyl, piperidinyl, morpholinyl, 4-methylpiperazinyl or 1,3-thiazolidinyl group.

2. Compound according to Claim 1, **characterized in that** X is in the 8 or 9 position and represents a hydrogen or halogen atom, Y represents a hydrogen atom, R₁ represents a methyl or ethyl group, R₂ represents a hydrogen atom or a methyl group, R₃ represents a methyl group, or else R₂ and R₃ form, with the nitrogen atom which carries them, an azetidinyl or pyrrolidinyl ring.

3. Medicament, **characterized in that** it is composed of a compound according to either of Claims 1 and 2.

4. Pharmaceutical composition, **characterized in that** it comprises a compound according to either of Claims 1 and 2, in combination with an excipient.
